# EUROPEAN PATENT SPECIFICATION

(11) **EP 2 973 100 B1**
(45) Date of publication and mention of the grant of the patent: **10.10.2018**
(21) Application number: 14720796.3
(22) Date of filing: 13.03.2014
(51) Int. Cl.: A61M 1/14, A61M 1/34, G05D 7/06, A61M 1/16, G16H 40/63, A61M 1/26, G06F 3/0484

(54) **EXTRACORPOREAL BLOOD TREATMENT FLUIDS INTERFACE**
FLUIDSCHNITTSTELLE FÜR EXTRAKORPORALE BLUTBEHANDLUNG
INTERFACE DE FLUIDES DE TRAITEMENT DU SANG EXTRACORPOREL

(30) Priority: 15.03.2013 US 201361793620 P
(43) Date of publication of application: 20.01.2016
(73) Proprietor: Gambro Lundia AB, 220 10 Lund (SE)
(72) Inventor: SCHAEFER, Jonas, St. Paul, MN 55106 (US); O'MAHONY, John, Maple Grove, MN 55311 (US); LENDWAY, Tom, Vadnais Heights, MN 55127 (US); LUCAS, Jonathan, Long Beach, CA 90804 (US); BERNARD, Steve, V., Eden Prairie, MN 55346 (US)
(74) Representative: Ponzellini, Gianmarco
(86) International application number: PCT/US2014/026215
(87) International publication number: WO 2014/151669

(56) References cited:
- WO-A2-2007/124069
- WO-A2-2007/144427
- WO-A2-2011/002853
- US-A1- 2008 027 368
- US-A1- 2012 109 037
- US-A1- 2012 185 267

## Description

### CROSS REFERENCE TO RELATED APPLICATION

This application claims the benefit of U.S. Provisional Patent Application Serial No. 61/793,620 filed on 15 March 2013 and entitled "Extracorporeal Blood Treatment Fluids Interface."

### BACKGROUND

The disclosure herein relates to extracorporeal blood treatment. More particularly, the disclosure relates to user interfaces for the display of status information related to fluids used in extracorporeal blood treatment.

Extracorporeal blood treatment may refer to taking blood from a patient, treating the blood outside the patient, and returning the treated blood to the patient. Extracorporeal blood treatment is typically used to extract undesirable matter or molecules from the patient's blood, and/or to add beneficial matter or molecules to the blood. Extracorporeal blood treatment may be used with patients incapable of effectively eliminating matter from their blood, for example, in the case of a patient who is suffering from temporary or permanent kidney failure. These and other patients may undergo extracorporeal blood treatment to add to or to eliminate matter from their blood, to maintain an acid-base balance or to eliminate excess body fluids, for instance.

In a variety of extracorporeal blood treatments, one or more fluids, or liquids, may be supplied to the extracorporeal blood treatment apparatus for use during the treatments and one or more fluids may be collected as a part of the treatments. Both the supplied and collected fluids may be stored in one or more reservoirs. Those reservoirs may, during the course of treatment of a single patient, need to be replaced as they are either emptied (in the case of fluids supplied as a part of the treatment) or are filled to capacity (in the case of fluids collected as a part of the treatment). US 2008/027368 and US 2012/109037 disclose an extracorporeal blood treatment apparatus with pumps and fluid lines having a display showing a representation of at least one container.

### SUMMARY

The present invention is related to systems according to claim 1 that provide graphical user interfaces that include a fluid region depicting various status information related to fluids used in extracorporeal blood treatments. One or more fluid areas may be defined, or depicted, in the fluid region of the graphical user interface. Each of fluid areas may include a pump element and reservoir element. The pump element may be representative of pumping apparatus of an extracorporeal blood treatment system, and the reservoir element may be representative of reservoir apparatus of an extracorporeal blood treatment system. Additionally, one or more notifications may be provided in the fluid region proximate one or more fluid areas to provide status information for each fluid. A user may glance at the graphical user interfaces described herein to be provided with useful status information such as, e.g., how much time remains before the next reservoir change must occur, etc.
One exemplary extracorporeal blood treatment system may include a display apparatus including a graphical user interface and a computing apparatus operatively coupled to the display apparatus. The graphical user interface may be configured to depict a fluid region and the computing apparatus may be configured to display on the graphical user interface a fluid region including one or more fluid areas. Each fluid area of the one or more fluid areas may correspond to a fluid. At least one fluid area of the one or more fluid areas may include a pump element and a reservoir element. The pump element may define a flow rate of the fluid and the reservoir element may include a fluid level representative of an amount of the fluid within a reservoir storing the fluid. The computing apparatus may be configured to display a next reservoir change notification proximate a fluid area of the one or more fluid areas configured to indicate that the fluid within a reservoir is the next fluid that requires a reservoir change. The next reservoir change notification may include a time period representing an amount of time left before the reservoir change is required.

One exemplary method for an extracorporeal blood treatment system may include displaying on a graphical user interface a fluid region including one or more fluid areas. Each fluid area of the one or more fluid areas may correspond to a fluid, and at least one fluid area of the one or more fluid areas may include a pump element and a reservoir element. The pump element may define a flow rate of the fluid and the reservoir element may include a fluid level representative of an amount of the fluid within a reservoir storing the fluid. The exemplary method may include displaying a next reservoir change notification proximate a fluid area of the one or more fluid areas configured to indicate that the fluid within a reservoir is the next fluid that requires a reservoir change. The next reservoir change notification may include a time period representing an amount of time left before the reservoir change is required.

One exemplary extracorporeal blood treatment system may include a display apparatus including a graphical user interface and a computing apparatus operatively coupled to the display apparatus. The graphical user interface may be configured to depict a fluid region, and the computing apparatus may be configured to display on the graphical user interface a fluid region including one or more fluid areas. Each fluid area of the one or more fluid areas may correspond to a fluid, and at least one fluid area of the one or more fluid areas may include a pump element and a reservoir element. The pump element may define a flow rate of the fluid. The computing apparatus may be further configured to display a notification proximate a fluid area of the one or more fluid areas configured to provide status information of the fluid within a reservoir.

One exemplary method for an extracorporeal blood treatment system may include displaying on a graphical user interface a fluid region including one or more fluid areas. Each fluid area of the one or more fluid areas may correspond to a fluid, and at least one fluid area of the one or more fluid areas may include a pump element and a reservoir element. The pump element may define a flow rate of the fluid. The exemplary method may further include displaying a notification proximate a fluid area of the one or more fluid areas configured to provide status information of the fluid within a reservoir.

In at least one example the reservoir element may include a fluid level representative of an amount of the fluid within a reservoir storing the fluid and a graphical depiction of a fluid stored in a fluid bag. The fluid level representative of the amount of fluid within the reservoir may be defined by the level of fluid graphically depicted in the fluid bag. In at least another example the reservoir element may include a fluid level representative of an amount of the fluid that has been pumped through the pump element of the fluid region.

In one or more examples the one or more fluid areas may include a plurality of fluid areas, wherein the plurality of fluid areas corresponds to one or more of pre blood pump, effluent, citrate, blood flow rate, patient fluid removal, dialysate, replacement fluid, anticoagulation, patient plasma loss, and calcium. Further, in one or more examples, the reservoir element may include a graphical depiction of a fluid stored in a fluid bag, and the fluid level representative of the amount of fluid within the reservoir may be defined by the level of fluid graphically depicted in the fluid bag.

In one or more examples, the computing apparatus may be further configured to execute or the method may further include displaying on the graphical user interface a change region, allowing a user to select the change region to initiate one of a flow rate change and a bag change, and displaying a time period representing an amount of time left before the reservoir change is required proximate a plurality of fluid areas of the one or more fluid areas. Further, in one or more examples, each fluid area of the one or more fluid areas may include a fluid connection extending between the pump element and the reservoir element.

In one or more examples, the computing apparatus may be further configured to execute or the method may further include displaying a stop notification proximate a fluid area of the one or more fluid areas when a pump for the fluid is paused, and dismissing the stop notification if the pump resumes operation.

In one or more examples, the computing apparatus may be further configured to execute or the method may further include displaying a changing reservoir notification proximate a fluid area of the one or more fluid areas when a reservoir for the fluid is being changed and dismissing the changing reservoir notification if the reservoir is removed.

In one or more examples, the computing apparatus may be further configured to execute or the method may further include displaying a calibration, or testing, notification (e.g., a weighing notification, a syringe calibration notification, etc.) proximate a fluid area of the one or more fluid areas when a reservoir for the fluid is being calibrated by the extracorporeal blood treatment system and dismissing the calibration notification if the reservoir is finished being calibrated and ready for use (e.g., the reservoir weighs greater than a selected value, etc.).

In one or more examples, the exemplary extracorporeal blood treatment system may further include one or more pumps configured to move at least one of blood and a treatment solution during extracorporeal blood treatment and one or more reservoir scales. Each reservoir scale of the plurality of reservoir scales may be configured to weigh a reservoir operably attached to the reservoir scale, and each fluid area of the one or more fluid areas may be representative of a pump of the one or more pumps configured to pump the fluid represented by the fluid area and one reservoir scale of the one or more reservoir scales configured to weigh the fluid. In at least one example, the exemplary extracorporeal blood treatment system may further include one or more reservoir status lights. Each reservoir status light of the one or more reservoir status lights may be associated with one reservoir scale of the one or more of reservoir scales, and the one or more reservoir status lights may include a first reservoir status light associated with a first reservoir scale of the one or more reservoir scales and a second reservoir status light associated with a second reservoir scale of the one or more reservoir scales. Further, the first reservoir status light may emit light from a location that is closer to the first reservoir scale than the second reservoir scale.

The above summary of the present disclosure is not intended to describe each embodiment or every implementation thereof. Advantages, together with a more complete understanding of the present disclosure, will become apparent and appreciated by referring to the following detailed description and claims taken in conjunction with the accompanying drawings.

### BRIEF DESCRIPTION OF THE DRAWING

FIG. 1 is a block diagram of an exemplary extracorporeal blood treatment system including input apparatus, display apparatus, and treatment apparatus that may utilize the user interfaces and methods described herein.
FIG. 2 is an illustration of an exemplary extracorporeal blood treatment system that may include graphical user interfaces as described herein.
FIGS. 3-9 are screenshots of graphical user interfaces including a fluid region depicting status information related to fluids for use in extracorporeal blood treatment systems, for example, such as shown generally in FIGS. 1-2.

### DETAILED DESCRIPTION OF EXEMPLARY EMBODIMENTS

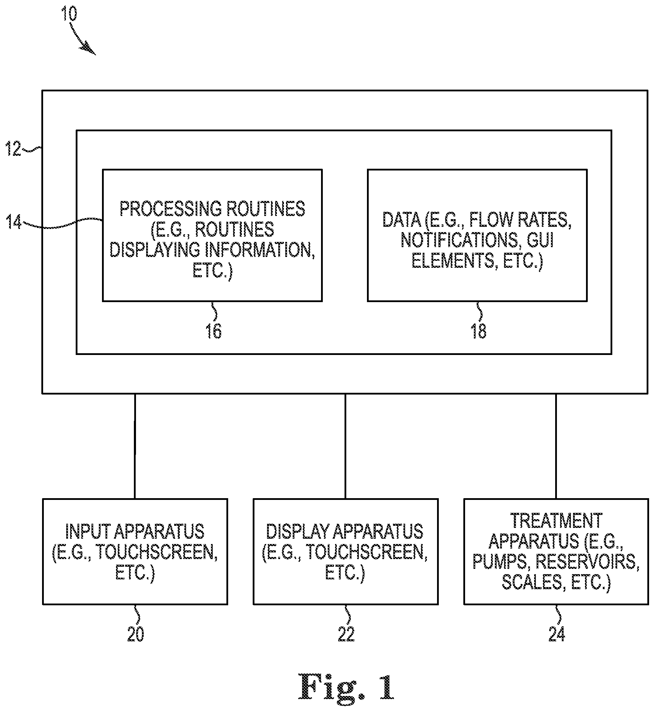

In the following detailed description of illustrative examples, reference is made to the accompanying figures of the drawing which form a part hereof, and in which are shown, by way of illustration, specific embodiments which may be practiced. It is to be understood that other embodiments may be utilized and structural changes may be made without departing from (e.g., still falling within) the scope of the disclosure presented hereby.
Exemplary systems and methods providing graphical user interfaces including a fluid area for use in an extracorporeal blood treatment shall be described with reference to Figures 1-9. It will be apparent to one skilled in the art that elements or processes from one embodiment may be used in combination with elements or processes of the other embodiments, and that the possible embodiments of such systems and methods using combinations of features set forth herein is not limited to the specific embodiments shown in the Figures and/or described herein. Further, it will be recognized that the embodiments described herein may include many elements that are not necessarily shown to scale. Still further, it will be recognized that timing of the processes and the size and shape of various elements herein may be modified but still fall within the scope of the present disclosure, although certain timings, one or more shapes and/or sizes, or types of elements, may be advantageous over others.

The exemplary systems and/or methods may include graphically displaying a fluid region depicting one or more fluid areas. Each fluid area may include a pump element, or flow rate button, and a reservoir element such as a reservoir bag. The pump elements and reservoir elements may be both colored to indicate the solution corresponding thereto. The pump elements may correspond to pumps operatively coupled to an extracorporeal blood treatment system and the reservoir elements may correspond to reservoirs also operatively coupled to the extracorporeal blood treatment system.

The reservoir elements may be displayed on a graphical user interface with volumes that closely match the actual volumes in the physical reservoirs themselves. As the actual reservoirs (e.g., bag reservoirs) empty and fill in the extracorporeal blood treatment system, the reservoirs on the graphical user interface may be displayed as emptying and filling.

During normal operation, the reservoir closest to needing attention may be continuously identified by a notification, or alert, to provide particular status information. For example, the notification may include a frame and a mask over the reservoir element displaying "Next Change" and a time until the state of the reservoir may cause the system to initiate an alarm. In this way, a user may be notified which of the reservoirs should be changed next, and when the reservoir change is required in order to maintain continuous therapy. By touching a change region, or button, an operator may have the ability to display the time until the next change is required for all reservoirs at once.

A user, or operator, may be able to adjust the amount of time remaining before they will be reminded to change a bag that is empty/full. For example, a user may select a remaining time amount before a bag change is required that will trigger a reminder, or alert. During normal operation, a yellow flashing "Attention" notification similar to the "Next Change" notification may be displayed proximate a reservoir element when the selected remaining time amount has been reached.

When a reservoir is being changed, a plurality of reservoir elements may be displayed above a dialog that describes the process of changing a single reservoir selected by a user. Reservoir elements may be displayed in the same arrangement as they were displayed during normal operations except that each reservoir element may include a next change notification and the selected reservoir element may be highlighted. During the reservoir change process, the state of the reservoir to be changed may be displayed as a notification using such labels such as "Changing," "No Bag," "Weighing," "Calibrating," and "Testing" in place of "Next Change."

The fluid areas of the exemplary graphical user interfaces may directly mimic the actual connections on and/or in an extracorporeal blood treatment system, and the association between the fluid areas and the actual physical components may allow a user to more easily understand the pumps and reservoirs as well as their relationship to the system overall. Further, by displaying reservoirs on the graphical user interface that drain and fill in a similar way as the actual physical reservoirs, a user may more easily accept feedback from the interface as being trustworthy. For example, when notification appears proximate a reservoir such as "Changing" or "No Bag," a user may receive positive feedback as they interact with the system. Additionally, the connection and association of the pump elements and the reservoir elements, as well as the notifications proximate thereto, and the various reservoir and notification "states" may create a user experience that more accurately reflects the system, may continuously instruct the user, and may provide a visual representation of the prescription being delivered to the patient.

An exemplary extracorporeal blood treatment system 10 depicted in FIG. 1 may be used to execute the exemplary methods and/or processes described herein. In at least one embodiment, the system 10 may be a machine for the extracorporeal treatment of blood. The system 10 could, for example, alternatively be a blood processing device or a blood component preparation device or other medical apparatus for fluid delivery/collection.

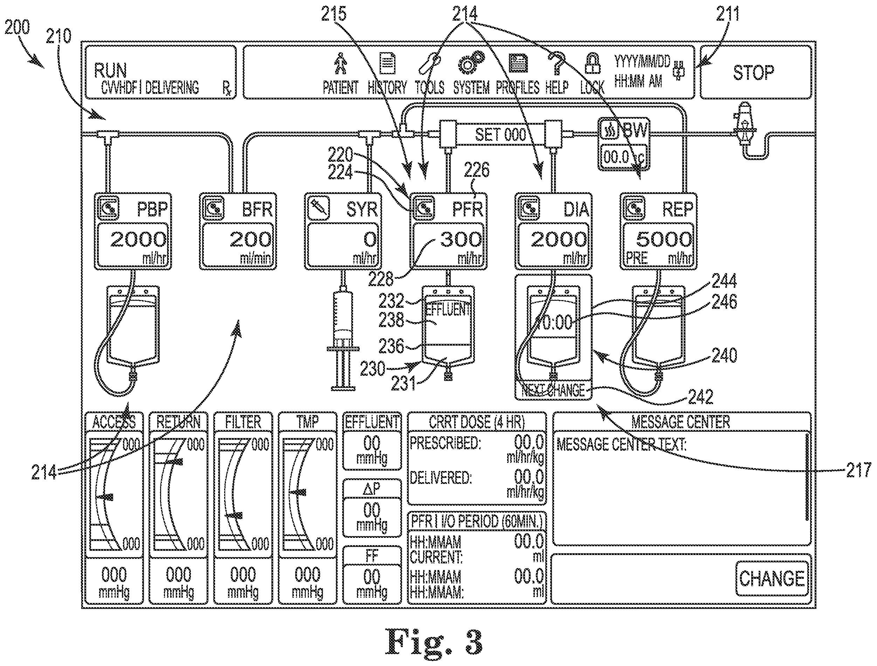

As shown, the exemplary extracorporeal blood treatment system 10 includes computing apparatus 12. The computing apparatus 12 may be configured to receive input from input apparatus 20 and transmit output to display apparatus 22. Further, the computing apparatus 12 may include data storage 14. Data storage 14 may allow for access to processing programs or routines 16 and one or more other types of data 18 that may be employed to carry out exemplary methods and/or processes for use in performing (e.g., running a treatment, exchanging reservoirs, notifying users of problems, displaying status information, etc.) extracorporeal blood treatments. For example, the computing apparatus 12 may be configured to display status information on the display apparatus 22 such as, e.g., one or more fluids areas including information corresponding to one or more fluids (e.g., which will be described further herein with respect to FIGS. 3-9).

The computing apparatus 12 may be operatively coupled to the input apparatus 20 and the display apparatus 22 to, e.g., transmit data to and from each of the input apparatus 20 and the display apparatus 22. For example, the computing apparatus 12 may be electrically coupled to each of the input apparatus 20 and the display apparatus 22 using, e.g., analog electrical connections, digital electrical connections, wireless connections, bus-based connections, etc. As described further herein, a user may provide input to the input apparatus 20 to manipulate, or modify, one or more graphical depictions displayed on the display apparatus 22 to select and view various status information related to one or more fluids in extracorporeal blood treatments.

Further, various devices and apparatus may be operatively coupled to the computing apparatus 12 to be used with the computing apparatus 12 to perform one or more extracorporeal procedures/treatments as well as the functionality, methods, and/or logic described herein. As shown, the system 10 may include input apparatus 20, display apparatus 22, and treatment apparatus 24 operatively coupled to the computing apparatus 12 (e.g., such that the computing apparatus 12 may be configured to use information, or data, from the apparatus 20, 22, 24 and provide information, or data, to the apparatus 20, 22, 24). The input apparatus 20 may include any apparatus capable of providing input to the computing apparatus 12 to perform the functionality, methods, and/or logic described herein. For example, the input apparatus 20 may include a touchscreen (e.g., capacitive touchscreen, a resistive touchscreen, a multi-touch touchscreen, etc.), a mouse, a keyboard, a trackball, etc. The input apparatus 20 may allow a user to select and view various status information corresponding to one or more fluids when used in conjunction with the display apparatus 22 (e.g., displaying a graphical user interface).

Likewise, the display apparatus 22 may include any apparatus capable of displaying information to a user, such as a graphical user interface, etc., to perform the functionality, methods, and/or logic described herein. For example, the display apparatus 22 may include a liquid crystal display, an organic light-emitting diode screen, a touchscreen, a cathode ray tube display, etc. As described further herein, the display apparatus 22 may be configured to display a graphical user interface that includes one or more regions such as a fluid region including status information corresponding to one or more fluids used in an extracorporeal blood treatment. For example, the graphical user interface displayed by the display apparatus 22 may include, or display, a fluid region including one or more fluid areas, each fluid area corresponding to a different fluid used in an extracorporeal blood treatment. Each of these fluid areas may be used by a user to view status information corresponding to the fluid such as flow rate, amount of fluid within a reservoir, an amount of time left before a reservoir change, etc. As used herein, a "region" of a graphical user interface may be defined as a portion of the graphical user interface within which information may be displayed or functionality may be performed. Regions may exist within other regions, which may be displayed separately or simultaneously. For example, smaller regions may be located within larger regions, regions may be located side-by-side, etc. Additionally, as used herein, an "area" of a graphical user interface may be defined as a portion of the graphical user interface located with a region that is smaller than the region it is located within.

The processing programs or routines 16 may include programs or routines for performing computational mathematics, matrix mathematics, standardization algorithms, comparison algorithms, or any other processing required to implement one or more exemplary methods and/or processes described herein. Data 18 may include, for example, fluid data, flow rates, fluid volumes, notifications, blood flow, fluid removal rates, CRC, target blood temperature, graphics (e.g., graphical elements, icons, buttons, windows, dialogs, pull-down menus, graphic areas, graphic regions, 3D graphics, etc.), graphical user interfaces, results from one or more processing programs or routines employed according to the disclosure herein, or any other data that may be necessary for carrying out the one and/or more processes or methods described herein.

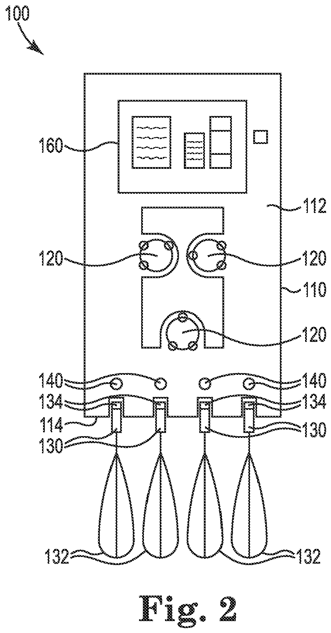

In one or more examples, the system 10 may be implemented using one or more computer programs executed on programmable computers, such as computers that include, for example, processing capabilities, data storage (e.g., volatile or non-volatile memory and/or storage elements), input devices, and output devices. Program code and/or logic described herein may be applied to input data to perform functionality described herein and generate desired output information. The output information may be applied as input to one or more other devices and/or methods as described herein or as would be applied in a known fashion.
The program used to implement the methods and/or processes described herein may be provided using any programmable language, e.g., a high level procedural and/or object orientated programming language that is suitable for communicating with a computer system. Any such programs may, for example, be stored on any suitable device, e.g., a storage media, that is readable by a general or special purpose program running on a computer system (e.g., including processing apparatus) for configuring and operating the computer system when the suitable device is read for performing the procedures described herein. In other words, at least in one example, the system 10 may be implemented using a computer readable storage medium, configured with a computer program, where the storage medium so configured causes the computer to operate in a specific and predefined manner to perform functions described herein. Further, in at least one example, the system 10 may be described as being implemented by logic (e.g., object code) encoded in one or more non-transitory media that includes code for execution and, when executed by a processor, is operable to perform operations such as the methods, processes, and/or functionality described herein.
Likewise, the system 10 may be configured at a remote site (e.g., an application server) that allows access by one or more users via a remote computer apparatus (e.g., via a web browser), and allows a user to employ the functionality according to the present disclosure (e.g., user accesses a graphical user interface associated with one or more programs to process data).
The computing apparatus 12 may be, for example, any fixed or mobile computer system (e.g., a controller, a microcontroller, a personal computer, mini computer, etc.). The exact configuration of the computing apparatus 12 is not limiting, and essentially any device capable of providing suitable computing capabilities and control capabilities (e.g., graphics processing, control of extracorporeal blood treatment apparatus, etc.) may be used.
As described herein, a digital file may be any medium (e.g., volatile or non-volatile memory, a CD-ROM, a punch card, magnetic recordable tape, etc.) containing digital bits (e.g., encoded in binary, trinary, etc.) that may be readable and/or writeable by computing apparatus 12 described herein.
Also, as described herein, a file in user-readable format may be any representation of data (e.g., ASCII text, binary numbers, hexadecimal numbers, decimal numbers, graphically, etc.) presentable on any medium (e.g., paper, a display, etc.) readable and/or understandable by a user.
In view of the above, it will be readily apparent that the functionality as described in one or more examples, according to the present disclosure may be implemented in any manner as would be known to one skilled in the art. As such, the computer language, the computer system, or any other software/hardware which is to be used to implement the processes described herein shall not be limiting on the scope of the systems, processes or programs (e.g., the functionality provided by such systems, processes or programs) described herein.
One will recognize that graphical user interfaces may be used in conjunction with the examples described herein. The graphical user interfaces may provide various features allowing for user input thereto, change of input, importation or exportation of files, or any other features that may be generally suitable for use with the processes described herein. For example, the graphical user interfaces may allow default values to be used or may require entry of certain values, limits, threshold values, or other pertinent information.
The methods and/or logic described in this disclosure, including those attributed to the systems, or various constituent components, may be implemented, at least in part, in hardware, software, firmware, or any combination thereof. For example, various aspects of the techniques may be implemented within one or more processors, including one or more microprocessors, DSPs, ASICs, FPGAs, or any other equivalent integrated or discrete logic circuitry, as well as any combinations of such components, or other devices. The term "processor" or "processing circuitry" may generally refer to any of the foregoing logic circuitry, alone or in combination with other logic circuitry, or any other equivalent circuitry.
Such hardware, software, and/or firmware may be implemented within the same device or within separate devices to support the various operations and functions described in this disclosure. In addition, any of the described components may be implemented together or separately as discrete but interoperable logic devices. Depiction of different features, e.g., using block diagrams, etc., is intended to highlight different functional aspects and does not necessarily imply that such features must be realized by separate hardware or software components. Rather, functionality may be performed by separate hardware or software components, or integrated within common or separate hardware or software components.
When implemented in software, the functionality ascribed to the systems, devices and methods described in this disclosure may be embodied as instructions and/or logic on a computer-readable medium such as RAM, ROM, NVRAM, EEPROM, FLASH memory, magnetic data storage media, optical data storage media, or the like. The instructions and/or logic may be executed by one or more processors to support one or more aspects of the functionality described in this disclosure.
The treatment apparatus 24 may include any apparatus used by an exemplary extracorporeal blood treatment system capable of performing extracorporeal blood treatments, such as, e.g., pumps, reservoirs, scales, treatment sets, filters, pressure sensors, etc. For example, the treatment apparatus 24 may include one or more elements, or components, of the extracorporeal blood treatment system 100 described herein with reference to FIG. 2.
The exemplary systems, and exemplary methods performed, or used, by such exemplary systems, described herein for the display of graphical user interfaces including a fluid area depicting status information corresponding to one or more fluids used in extracorporeal blood treatments may be generally referred to as dialysis systems. The general term dialysis as used herein includes hemodialysis, hemofiltration, hemodiafiltration, hemoperfusion, liver dialysis, and therapeutic plasma exchange (TPE), among other similar treatment procedures. In dialysis generally, blood is taken out of the body and exposed to a treatment device to separate substances therefrom and/or to add substances thereto, and is then returned to the body. Although extracorporeal blood treatment systems capable of performing general dialysis (as defined above, including TPE) shall be described herein with reference to the exemplary extracorporeal blood treatment system of FIG. 2, other systems such as those for infusion of drugs, performance of continuous renal replacement therapy (CRRT), extracorporeal membrane oxygenation (ECMO), hemoperfusion, liver dialysis, apheresis, TPE, etc. may benefit from the systems, methods, and apparatus described herein and the present disclosure is not limited to any particular fluid processing system.

Referring to FIG. 2, one illustrative example of an extracorporeal blood treatment system, or apparatus, 100 is depicted. The system 100 includes a housing 110 having a front face 112. The system further includes one or more pumps 120 used to move liquids through the apparatus as part of a treatment process. Although the pumps 120 are depicted in the form of peristaltic pumps, the pumps used in the extracorporeal blood treatment system described herein may be provided in a variety of alternative forms, e.g., piston pumps, pumps for use with syringes, diaphragm pumps, etc. In at least one example, pinch valves may further be used to control flow rates of fluids.
The extracorporeal blood treatment system 100 also includes, in one or more examples, a display 160 used to convey information to a user. The display 160 may also serve as an input device if, e.g., the display 160 is in the form of a touchscreen. Also, although the display 160 is depicted as being located in the housing 110, in one or more alternate examples, the display 160 may be separate from the housing 110 of the extracorporeal blood treatment system 100.
The extracorporeal blood treatment system 100 also includes reservoir scales 130, each of which is configured to hold and weigh a reservoir 132. The reservoir scales 130 are positioned below a bottom end 114 of the housing 110, at least in part because the reservoirs 132 are typically attached to and hang from the reservoir scales 130. Although the depicted example of extracorporeal blood treatment system 100 includes four reservoir scales 130 and associated reservoirs 132, alternative examples of an extracorporeal blood treatment apparatus as described herein may include one or more reservoir scales 130 and associated reservoirs 132 such as, e.g., as few as two reservoirs scales 130 and associated reservoirs 132, four or more reservoirs scales 130 and associated reservoirs 132, etc. In still other variations, one of the reservoir scales 130 may be used to hold and weigh two or more reservoirs 132 rather than a single reservoir 132 as depicted in FIG. 2.

In the example shown, the reservoirs 132 may be in the form of, e.g., flexible polymeric bags configured to hold liquids. Reservoirs 132, however, used in connection with the exemplary extracorporeal blood treatment systems described herein may take any suitable form in which liquids can be stored, e.g., bottles, tanks, cartons, syringes, jugs, etc.
The extracorporeal blood treatment system 100 depicted in FIG. 2 may also include passive color indicators 134 on each of the reservoir scales 130. The passive color indicators 134 may be used as a designation of the contents of each of the reservoirs 132 attached to the reservoir scale 130. For example, if one of the reservoirs 132 is connected to the extracorporeal blood treatment system 100 to collect waste fluid from, e.g., a dialysis filter, the passive color indicator 134 associated with the reservoir scale 130 holding the waste reservoir 132 may have a selected color that is different than, e.g., a reservoir scale 130 holding a reservoir 132 that is used to supply dialysate liquid within the same extracorporeal blood treatment system 100. The passive color indicators 134 used in connection with an extracorporeal blood treatment system as described herein may be in the form of patches, stickers, paint, or any other suitable technique of displaying a color to a user of the extracorporeal blood treatment system that does not involve emitting light. Although the passive color indicators 134 are depicted as being located on the reservoir scales 130, the passive color indicators 134 may, in one or more examples, be located on the housing 110, while in one or more other embodiments passive color indicators may be located on both the reservoir scales 130 and the housing 110.
A plurality of reservoir status lights 140 are also depicted in connection with the extracorporeal blood treatment system 100 of FIG. 2 and may be used to monitor the status of the reservoirs 132 attached to the reservoir scales 130 associated with the reservoir status lights 140. In one or more examples, the reservoir status lights 140 are located below the one or more pumps 120 and the display 160 of the extracorporeal blood treatment system 100. Because, in one or more examples, the reservoirs 132 hang from the reservoir scales 130, the reservoir status lights 140 may be described as being located below the one or more pumps 120 and above the reservoirs 132 attached to the reservoir scales 130 of the extracorporeal blood treatment system 100. Further, although the reservoir status lights 140 are depicted as being located on the front face 112 of the housing 110, the reservoir status lights 140 may, in one or more alternative examples, be provided on the reservoir scales 130 and/or on other surfaces of the extracorporeal blood treatment system 100. In such an example, one or more passive color indicators 134 associated with each of the reservoir scales 130 may be located on the housing 110 and/or on the reservoir scales 130.

Each of the reservoir status lights 140 may be associated with only one reservoir scale 130 of the extracorporeal blood treatment system 100. For example, a first reservoir status light 140 may be associated with a first reservoir scale 130, while a second reservoir status light 140 may be associated with a second reservoir scale 130. Although the depicted extracorporeal blood treatment system 100 includes only one reservoir status light 140 associated with each reservoir scale 130, in one or more alternative embodiments, two or more reservoir status lights 140 may be associated with one reservoir scale 130.
The display 160 may be used to monitor the operation of the extracorporeal blood treatment system 100 as well as the status of any reservoirs 132 attached to the reservoir scales 301 as described herein with reference to FIGS. 3-9. In the extracorporeal blood treatment system 100 described herein, the reservoir status lights 140 can, in one or more examples, be used to provide an indication of the status of a reservoir 132 attached to the reservoir scale 130 that is associated with a reservoir status light 140. For example, a reservoir status light 140 associated with a selected reservoir scale 130 is located closer to the selected reservoir scale 130 than any other reservoir scale 130 provided in the extracorporeal blood treatment apparatus. In the case of a first reservoir status light 140 associated with a first reservoir scale 130 and a second reservoir status light 140 associated with a second reservoir scale 130, the first reservoir status light 140 emits light from a location that is closer to the first reservoir scale 130 than the second reservoir scale 130.

In one or more examples, the reservoir status light 140 associated with a reservoir scale 130 may provide an indication that a reservoir 132 attached to the reservoir scale 130 has passed a selected weight limit as a part of monitoring the status of the reservoirs. That selected weight limit may, in the case of a reservoir 132 used to collect liquids from the extracorporeal blood treatment apparatus, be an upper limit such that passing (e.g., reaching and/or exceeding) the selected weight limit is an indication that the reservoir 132 is reaching or has reached its loading capacity and may need to be replaced with a reservoir 132 having more capacity to collect liquid. In the case of a reservoir 132 used to supply liquids to the extracorporeal blood treatment apparatus, the selected weight limit may be a lower limit such that passing (e.g., reaching and/or falling below) the selected weight limit is an indication that the reservoir 132 is reaching or has reached a level at which the reservoir 132 may need to be replaced with a fresh reservoir 132 containing additional liquid to be supplied to the extracorporeal blood treatment system 100.
As shown in FIG. 1, the treatment apparatus 24 may be operatively coupled, or connected, to the computing apparatus 12. Among the treatment apparatus 24 operably coupled to the computing apparatus 12 are the pumps 120 and reservoir scales 130 as shown in FIG. 2. Also, among the treatment apparatus 24 operably coupled to the computing apparatus 12 are the reservoir status lights 140.

The computing apparatus 12 may, in one or more examples, be configured to receive a weight signal from each reservoir scale 130, with the weight signal from each reservoir scale 130 being indicative of the weight of a reservoir 132 attached to the reservoir scale 130. The computing apparatus 12 may further be configured to make a determination that the reservoir 132 attached to the reservoir scale 130 from which the weight signal has been received has passed a selected weight limit at least partially based on the weight signal received from the reservoir scale 130. As discussed herein, the selected weight limit may be an upper limit or a lower limit depending on whether the reservoir is used to supply liquid or collect liquid from the extracorporeal blood treatment system 100. If the computing apparatus 12 makes a determination that a reservoir 132 associated with a reservoir scale 130 has passed the selected weight limit, the computing apparatus 12 may further be configured to change a mode of light emitted by the reservoir status light 140 associated with the reservoir scale 130 (e.g. varying the intensity of the light, changing the brightness of the light, turning the light on and off such that it blinks, blinking the light at one or more different rates, changing the color of the light, etc.) and/or to display various status information such as notifications on the graphical user interface.
The computing apparatus 12 is described herein as being, in one or more examples, configured to make a determination that the reservoir 132 attached to the reservoir scale 130 from which the weight signal has been received has passed a selected weight limit at least partially based on the weight signal received from the reservoir scale 130. That determination may be described as being made "at least partially based on the weight signal" because, in one or more examples, the determination that a weight limit has been passed and/or that a reservoir requires attention or replacement may be based on factors in addition to the weight signal, e.g., flow into or out of a reservoir 132 as measured using a pump 120 or other component of the extracorporeal blood treatment apparatus, etc.
The reservoir scales used to hold and weigh reservoirs used in the extracorporeal blood treatment apparatus described herein may take any number of a variety of different forms. Examples of some potentially suitable reservoir scales and associated structure may be found in International Publication WO 2004/069311 and US Patent 7,891,625, as well as the reservoir scales and hangers used in some commercially available hemodialysis machines (e.g., PRISMAFLEX machines available from Gambro Lundia AB, etc.).

In one or more examples, the reservoir scales used in the extracorporeal blood treatment apparatus described herein may have a loading position and an operating position. In particular, the reservoir scales may have a loading position in which it is easier or more convenient to remove and/or attach a reservoir to the reservoir scale and an operating position in which the reservoir scale is in a position and/or location that provides for accurate weighing of a reservoir attached to the reservoir scale. Screenshots depicting exemplary graphical user interfaces for use in displaying status information corresponding to one or more fluids used in extracorporeal blood treatments are depicted in FIGS. 3-8. Such exemplary graphical user interfaces may be depicted by the display apparatus 22 of the system 10 described herein with reference to FIG. 1 and/or the display screen 160 of the system 100 of FIG. 2. Additionally, the graphical user interfaces described herein may be depicted on a touchscreen, and in such configuration, the input apparatus would also be the touchscreen.
An exemplary graphical user interface 200 is depicted in FIG. 3 that may be generally used during and/or for the execution of an extracorporeal blood treatment. The graphical user interface 200 may include, among other regions, a fluids region 210. As shown, the fluids region 210 is depicted below a toolbar region 211 and extending from a left side to a right side of the graphical user interface 200. The fluids region 210 may include, or graphically depict, one or more fluid areas 214. Each of the fluid areas 214 may correspond, or represent, a fluid used in the extracorporeal blood treatment.

For example, as shown in FIG. 3, a Continuous Veno Venous Hemodiafiltration (CVVHDF) treatment is being presently executed, and thus, fluid areas 214 corresponding to the CVVHDF treatment are depicted in the fluids region 210 such as, e.g., pre-blood pump (PBP), blood flow rate (BFR), anticoagulation (SYR), patient fluid removal (PFR), dialysate (Dia), and replacement fluid (REP). Although a CVVHDF process is depicted in FIG. 3, the graphical user interface 200 may be configured for a plurality of different treatment processes such as, e.g., continuous ultrafiltration (SCUF), continuous veno-venous hemofiltration, (CVVH), continuous veno-venous hemodialysis (CVVHD), therapeutic plasma exchange (TPE), hemofiltration HP, hemoperfusion, continuous renal replacement therapy (CRRT), molecular adsorbent recirculating system (MARS), etc. More specifically, for each different treatment process, the graphical user interface 200 may include a fluids region 210 that includes fluid areas 214 corresponding to the one or more different fluids or fluid configurations used in each different treatment process. Although, six different fluid areas 214 are depicted in the fluids region 210 as shown, it is to be understood that the fluids region 210 may display, or include, more than six fluid areas 214 or less than six fluid areas 214 depending on the treatment being executed. For example, the one or more fluid areas 214 may include fluid areas corresponding to one or more of pre blood pump, effluent, citrate, blood flow rate, patient fluid removal, dialysate, replacement fluid, anticoagulation, patient plasma loss, calcium, etc. Further, although the fluid areas 214 are described herein as being "fluid" areas corresponding to different fluids, it is to be understood that each fluid area 214 may not always describe a different "fluid," and instead, may describe the same fluid along a different portion of the extracorporeal blood treatment fluid circuit.

Each fluid area 214 may include one or more portions to be configured to display status information related, or corresponding, to a fluid used in the extracorporeal blood treatment. Although many fluid areas 214 are similar, each fluid area 214 may be different from the next fluid area 214. For example, some fluid areas 214 may include more or less portions than other fluids areas depending on the type and amount of information to be conveyed, e.g., depending on the type of fluid, depending on where the fluid area is located with the extracorporeal blood treatment system, etc.

One or more fluid areas 214 may include a pump element and a reservoir element. The pump element of a fluid area 214 may include a graphical depiction, or icon, loosely depicting an actual pump, an acronym, or abbreviation, of the fluid, and a numerical field typically displaying the pump's flow rate. Different types of pumps elements may be displayed in each fluid area 214 depending on the therapy in use, and the pump elements themselves may change depending on their state and as a user interacts with them.

Exemplary PFR fluid area 215 will be further described herein. It is to be understood that other fluid areas 214 may include more or less portions and/or elements than the exemplary PFR fluid area 215, and that the exemplary PFR fluid area 215 is simply one example of a fluid area 214.

The PFR fluid area 215 may include a pump element 220 and a reservoir element 230. The pump element 220 may correspond to a physical pump such as, e.g., one of the pumps 120 of the extracorporeal blood treatment system 100 described herein with respect to FIG. 2. The pump element 220 may include a pump icon, or graphical representation 224 located in the upper left corner of the pump element 220. The pump icon 224 may be a graphical depiction of the type of pump used for the fluid, in this case PFR, corresponding to the PFR fluid area 215. Additionally, the pump element 220 may include an identifier portion 226 located in the upper right corner of the pump element 220 that may alphanumerically describe, or depict, the fluid corresponding to the PFR fluid area 215, which in this case, is "PFR."

The pump element 220 further includes a flow rate portion 228 depicted below the pump icon 224 and the identifier portion 226. The flow rate portion 228 may alphanumerically describe, or depict, the flow rate of the fluid corresponding to the PFR fluid area 215. Although the flow rate of the pump element 220 of the PFR fluid area is presently "300" milliliters per hour (m/1) as shown in FIG. 3, the flow rate may change depending on the treatment and/or phase within the treatment.

The reservoir element 230 may correspond to a physical reservoir such as, e.g., one of the reservoirs 132 of the extracorporeal blood treatment system 100 described herein with respect to FIG. 2. The reservoir element 230 may be configured to depict a fluid level representative of an amount of the fluid within a reservoir storing the fluid corresponding to the PFR fluid area 215. The fluid level 236 may be shown in many ways. As shown, the reservoir element 230 includes a graphical depiction of a fluid 231 (in this case, PFR) stored in a fluid bag 232 and the fluid level 236 representative of the amount of fluid within the reservoir is defined by the fluid 231 graphically depicted in the fluid bag 232.

The reservoir element 230 may include a depiction of the physical type of reservoir being used for the fluid in the extracorporeal blood treatment system. For example, as shown, the reservoir element 230 of the PFR fluid area 215 is a graphical depiction of a fluid bag 232, which may correspond to the physical bag reservoir being used for PFR in the exemplary extracorporeal blood treatment system. As described herein, the fluid level 236 may correspond to the amount of fluid within the physical reservoir of the extracorporeal blood treatment system, and as such, a user may glance at the graphical user interface 200 to determine the volume of one or more reservoirs (e.g., how empty a reservoir is, how full a reservoir is, etc.) based on the fluid levels 236 of the fluid areas 214 without looking at the physical reservoir.

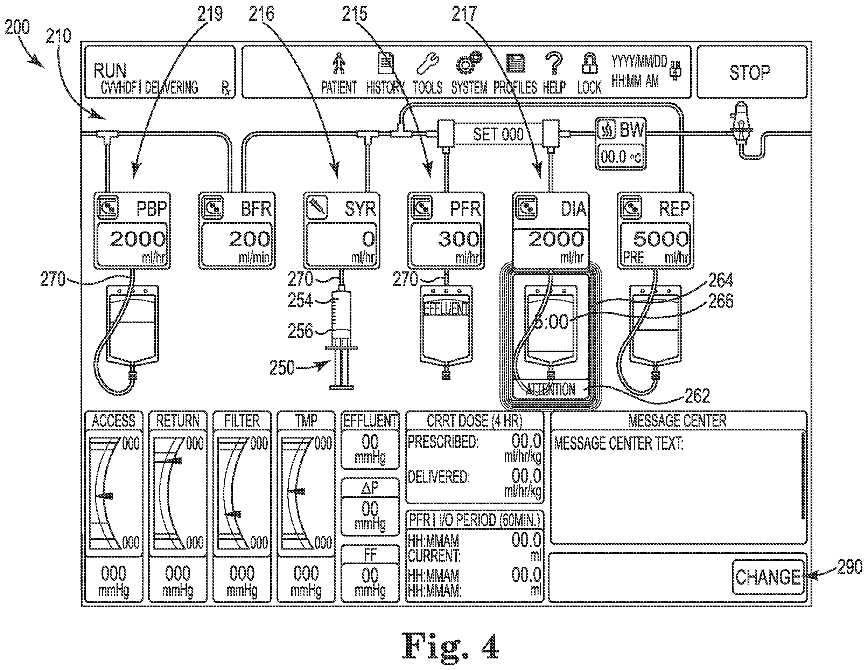

The reservoir elements of the fluid areas 214 may depict types of reservoirs other than fluid bags such as, e.g., cylinders, jugs, syringes, flasks, etc. For example, as shown in anticoagulation (SYR) fluid area 216 identified in FIG. 4, the reservoir element 250 depicts a syringe that includes a fluid (e.g., anticoagulation fluid) 254 and the plunger position (adjacent the fluid) depicts the fluid level 256 representative of the amount of fluid 254 within the syringe.

In at least one example, the fluid levels 236, 256 may be representative of an amount of fluid that has been pumped, or moved, through the pump element 220.
For example, therapeutic plasma exchange (TPE) the total volume of one or more fluids that have been has been pumped, or moved, through the pump element 220 may be displayed, or graphically depicted, using the fluid levels 236, 256. In other words, the total fluid volume delivered through the course of the treatment may be depicted. Further, in one or more examples, a user may selected whether the fluid levels 236, 256 indicate the present volume, or amount, of fluid within a reservoir and/or the total volume, or amount, of fluid that has been pumped, or moved, through a pump.
The reservoir element 230 may further include an alphanumeric description 238 since more than one different type of fluid may be assocated with PFR. As shown in FIG. 3, the alphanumeric description 238 recites "EFFLUENT" to indicate that the fluid 231 filling the PFR reservoir is effluent.
The graphical user interface 200 may further include one or more notifications located proximate one or more of the fluid areas 214 to, e.g., convey, or depict, status information corresponding to the one or more fluids that a user may glance to determine the status of the exemplary extracorporeal blood treatment system. Often, the notifications may provide an indication to a user of what task may need to be executed by the user and what time the task may need to be executed. The one or more notifications may include next reservoir change notifications, attention notifications, changing notifications, no bag notifications, calibration notifications (e.g., weighing notifications, syringe calibration notifications, etc.), new bag notifications, stop notifications, etc.
For example, a next reservoir change notification 240 is depicted proximate the dialysate (Dia) fluid area 217 as shown in FIG. 3. The next reservoir change notification 240 indicates that the fluid, in this case, dialysate, within the dialysate reservoir is the next fluid that requires a reservoir change. As such, the user may glance at the graphical user interface 200 and ascertain that the next task may be to change the dialysate reservoir based on the next reservoir change notification 240.

Further, as shown in FIG. 3, only one next reservoir change notification 240 is depicted among the fluids areas 214, or in the fluids region 210, so as to only depict the next reservoir change.

The next reservoir change notification 240 includes one or more graphical elements or components. As shown, the next reservoir change notification 240 includes an identifier portion 242, a border 244, and a time remaining portion 246. The identifier portion 242 may include an alphanumeric identifier such as, in this example, "Next Change," to indicate the type of notification. The border 244 extends around the reservoir element of the fluid area 217 to, e.g., show that the notification 240 pertains to the reservoir. In other notifications, the border 244 may extend around the pump element to, e.g., show that the notification pertains to the pump.

The time remaining portion 246 includes a time period representing an amount of time left before a reservoir change is required. As shown in FIG. 3, 10 minutes, and 0 seconds is remaining before a reservoir change for dialysate is required. As the amount of time left before the reservoir change is required decreases, the reservoir change may become more imminent to maintain the presently executing extracorporeal blood treatment. As such, it may be more important to acquire the attention of a user to change the reservoir. To do so, the graphical user interface 200 may include an attention notification 260 as shown in FIG. 4.

Similar to the next reservoir change notification 240, the attention notification 260 may include an identifier portion 262, a border 264, and a time remaining portion 266. The identifier portion 262 identifies the notification 260 as an "Attention" notification, and the border 264 extends around the reservoir element of the fluid area 217 to, e.g., show that the notification 260 pertains to the reservoir. In this example, because the attention notification 260 is more imminent than the next bag change notification 240, the border 264 may flash or depict any other attention "grabbing" graphical animation. Similar to the next bag change notification 240, the time remaining portion 266 may include a time period representing an amount of time left before the reservoir change is required.

The one or more exemplary notifications to be displayed on the graphical user interface 200 as described here may correspond to the reservoir status lights 140 described herein with reference to the system 100 of FIG. 2. For example, one or more of the reservoir status lights 140 may correspond to the next bag change notification 240 or the attention notification 260 such that, e.g., a user may glance at either the graphical user interface 200 or the reservoir status lights 140 to determine the status of one or more fluids used in the extracorporeal blood treatment. In at least one example, when an next reservoir change notification 240 is displayed proximate a fluid area 214, the corresponding reservoir status lights 140 proximate the reservoir represented by the particular fluid area having the next bag change notification 240 may flash, or blink, yellow to, e.g., signify "next reservoir change." In at least one example, when an attention notification 260 is displayed proximate a fluid area 214, the corresponding reservoir status lights 140 proximate the reservoir represented by the particular fluid area having the attention notification 260 may flash, or blink, red to, e.g., signify "attention."
Each of the pump element and reservoir element of the fluid areas 214 may be depicted as being interconnected. For example, fluid connections 270 may extend between the pump element and reservoir element of one or more of the fluids areas 214 as shown in FIG. 4. The fluid connection 270 of each fluid area 214 may accurately depict the physical fluid connection between the reservoir and pump of the extracorporeal blood treatment system. Additionally, the reservoir may be used to collect fluid from the patient and/or supply fluid to be used in the blood treatment process (e.g., delivered to the patient), and depending on the functionality of the reservoir, the fluid connection 270 depicted may be different. For example, as shown in FIG. 4, the fluid connection 270 for the PBP fluid area 219 is shown extending from the bottom of the reservoir element (e.g., a bag reservoir) to the pump element, which may indicate that the PBP reservoir stores and supplies fluid to be used in the blood treatment process. The fluid connection 270 for the PFR fluid area 215 is shown extending from the top of the reservoir element (e.g., a bag reservoir) to the pump element, which may indicate that the PFR reservoir stores and collects fluid from the patient.

To change a reservoir such as, e.g., the dialysate reservoir, a user may select a change region 290 as shown in FIG. 4. To select the change region 290, a user may use input apparatus 20 of the exemplary extracorporeal blood treatment system 10 described herein with reference to FIG. 1. For example, the input apparatus 20 may be a touch screen that corresponds to the graphical user interface 200. As used herein, when a user "selects" a region or area of the graphical user interface, it is to be understood that selecting the region or area may be conducted in many different ways using many different types of input apparatus. For example, when the input apparatus is a touch screen, a user may select a region or area by "touching" the region or area with their finger or using a pointing device such as a stylus. Further, for example, when the input apparatus is a mouse or similar pointing device, a user may select a region or area by locating an arrow or cursor over the desired region or area "clicking" the region or area. Still further, for example, when the input apparatus is a series of buttons and/or knobs, a user may select a region or area by using the buttons and/or knobs to navigate to the region or area and selecting it by depressing a button and/or knob. In this example, a user may touch the change region 290, which graphically depicts a button.

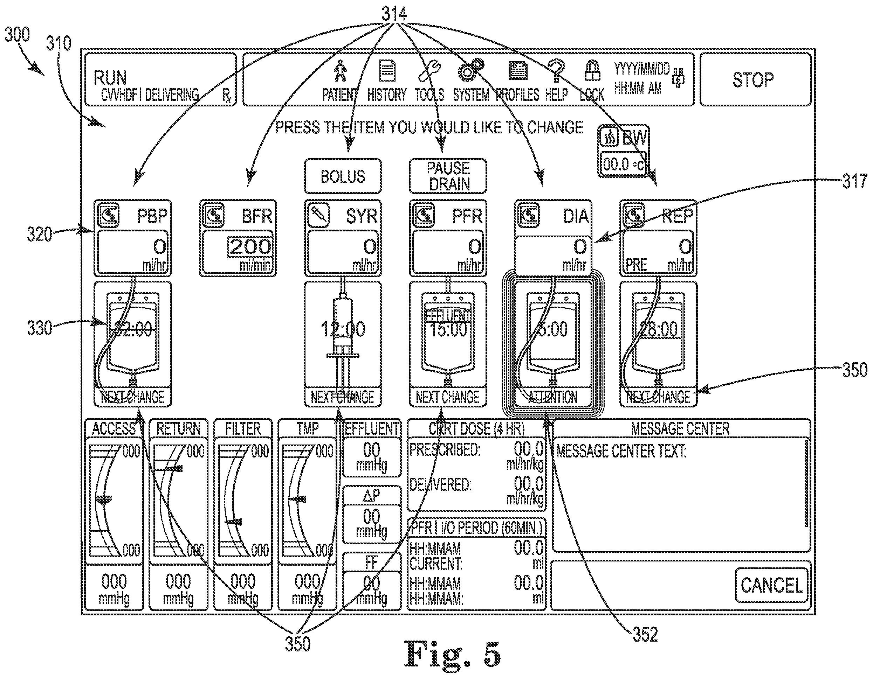

After a user has selected the change region 290 of FIG. 4, the graphical user interface 300 of FIG. 5 may be depicted. The graphical user interface 300 of FIG. 4 may include a fluids region 310 similar to the fluids region 210 described herein with reference to FIGS. 3-4. For example, the fluids region 310 may include one or more fluid areas 314, with each fluid area including a pump element 320 and a reservoir element 330.

The graphical user interface 300 may further include a plurality of next reservoir change notifications 350 for a plurality of the fluid areas 314 and an attention notification 352 for the fluid area 314 requiring the most imminent reservoir change. In other words, more than one notification such as reservoir change notifications 350, attention notification 352, etc. may be depicted in the fluids region 310. In this example, the dialysate fluid area 317 has the attention notification 352 and has the least amount of time remaining before a required reservoir change out of the remaining fluid areas 314.

A user may initiate a reservoir change sequence by selecting one of the fluids areas 314 that the user will be changing. For example, if a user would like to initiate a reservoir change for the dialysate, the user may select the dialysate fluid area 317 by, e.g., selecting the pump element, selecting the reservoir element, etc.

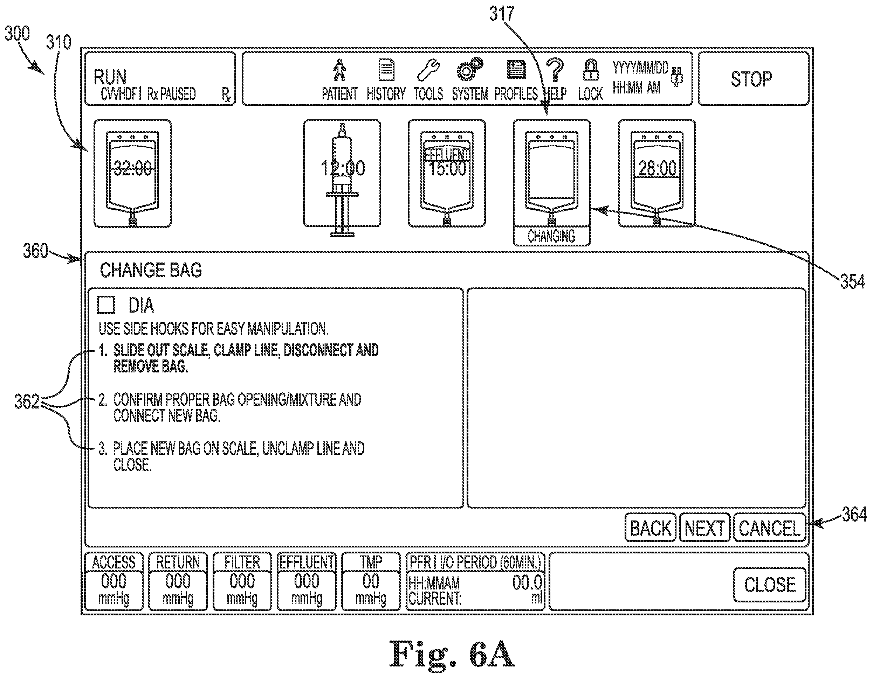

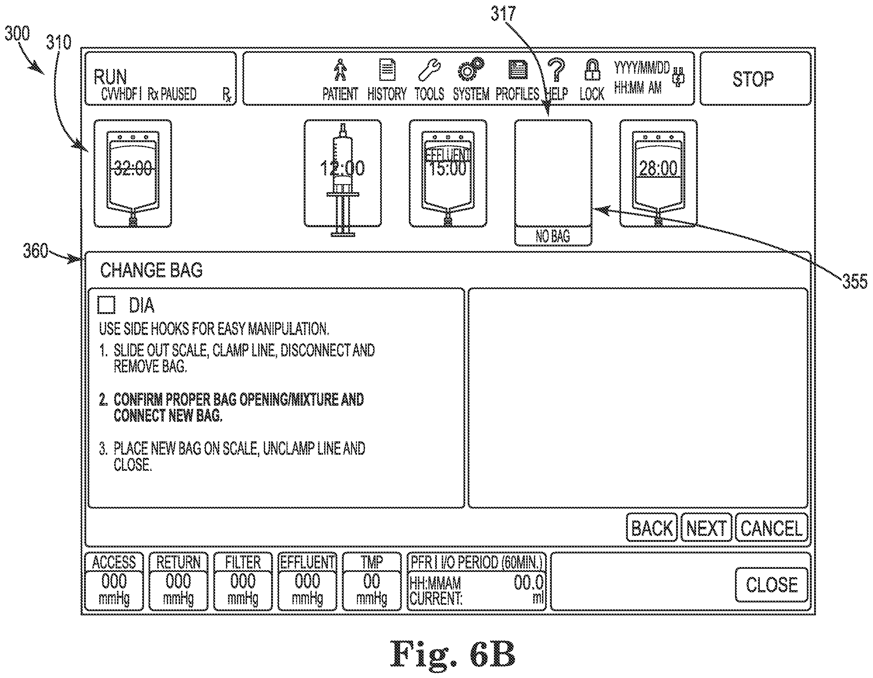

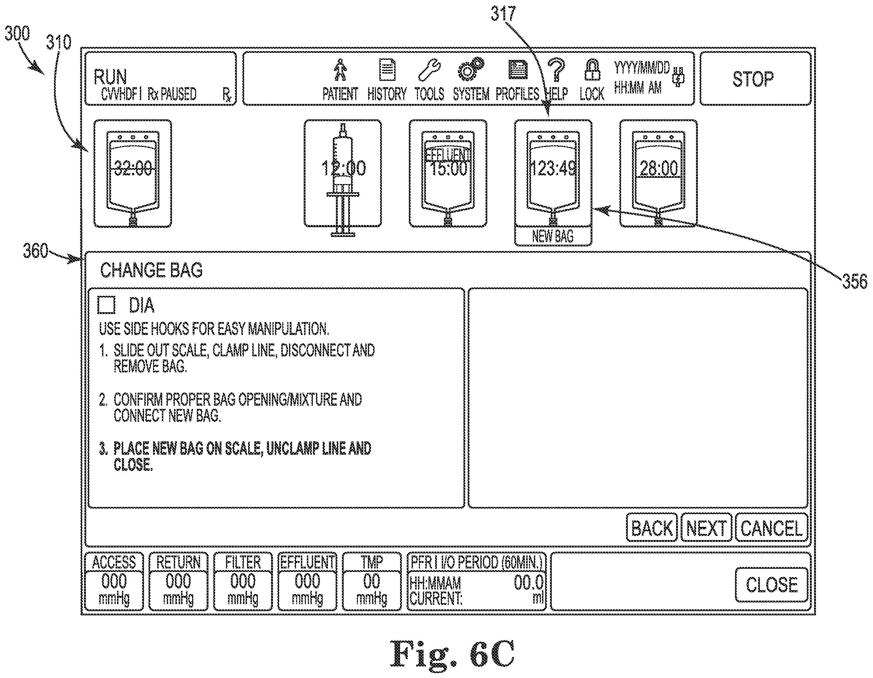

In at least one example, instead of a plurality of next reservoir change notifications 350 being displayed for a plurality of the fluid areas 314, a change region, or button, may be displayed over or proximate each of the plurality of fluid areas 314, which may be selected to change the associated reservoir, and a time remaining portion may be displayed proximate each fluid area including a time period representing an amount of time left before a reservoir change is required.
After a user has selected the dialysate fluid area 317 or initiated a reservoir change manually by, e.g., physically removing a reservoir or pulling out a scale, a reservoir change instruction region 360 may be displayed below the fluids region 310 in the graphical user interface 300 as shown in FIG. 6A. The reservoir change instruction region 360, as shown, may be a "pop-over" window (e.g., a graphical element that is located over the remainder of the graphical user interface 300). The reservoir change instruction region 360 may include a list of steps 362 for changing the reservoir and navigation areas, or buttons, 364 to traverse the steps 362.
Additionally, a changing reservoir notification 354 may be depicted proximate the fluid area 317 while the reservoir is being changed. As shown, the changing reservoir notification 354 includes a border similar to the border 244 and an identifier portion 242 similar to the next reservoir change notification 240 described herein with reference to FIG. 3. In at least one example, the reservoir change instruction 360 may be an operation specific graphical user interface and may include a graphical depiction of only the fluid area and/or reservoir being changed.
As a user progresses through the steps 362 of the reservoir change, a notification proximate the fluid reservoir being changed may change to, e.g., indicate the present status of the reservoir. For example, after a user has slid out the scale using a reservoir changing/hold structure, clamped the line to the reservoir, disconnected the reservoir, and removed the reservoir as instructed in the first step 362 and used the navigation areas 364 to traverse to the next step, a no reservoir, or no bag, notification 355 may be located proximate the fluid area 317 as shown in FIG. 6B. In other words, after the reservoir has been removed from the system, the changing reservoir notification 354 may be dismissed and replaced by a no reservoir notification 355. Further, for example, after a user has confirmed a proper reservoir, or bag, opening/mixture and connected the new reservoir as instructed in the second step 362 and used the navigation areas 364 to traverse to the next step, a new reservoir, or new bag, notification 356 may be located proximate the fluid area 317 as shown in FIG. 6C. In other words, after a new reservoir has been attached to the system, the no reservoir notification 355 may be dismissed and replaced by a new reservoir notification 356.

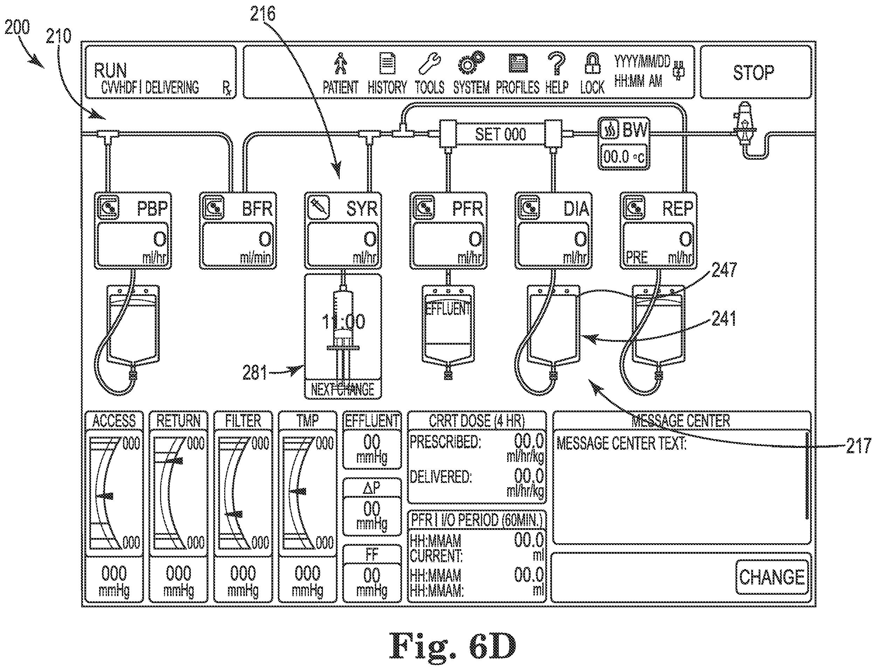

After a user has completed the last step, e.g., placed the new reservoir, or bag, on the scale, unclamped the line, and closed or slid the scale back using the reservoir changing/hold structure, the user may use the navigation areas 364 and select "Next" which may display the graphical user interface 200 as shown in FIG. 6D. As shown, the reservoir element 241 of the dialysate fluid area 217 indicates that the dialysate reservoir is full as shown by the fluid level 247. Additionally, as shown in FIG. 6D, a next reservoir change notification is no longer proximate the dialysate fluid area 217, and instead, a next reservoir change notification 281 may be depicted, or displayed, proximate anticoagulation (SYR) fluid area 216 (which is the next reservoir requiring a reservoir change).

Although not shown, after a new reservoir has been located on, or attached to, the system, the system may be configured to weigh the new reservoir. While the system is weighing the reservoir, a calibration notification such as a weighing notification may be displayed proximate the fluid area 317, or on any other area of the graphical user interface 200, that recites "Weighing," "Testing," and/or "Calibrating." While a reservoir is being weighed, a user may not able to proceed until the system completes weighing the reservoir and the weight of the reservoir meets one or more requirements. For example, a new reservoir may need to conform to a particular, or selected, weight requirement. As such, unless the new reservoir conforms to the weight requirement, a user may not be allowed to proceed with the blood treatment.

If the weight requirement is not met, the weighing notification may not be dismissed and/or another notification may be presented indicating that the new reservoir has not met the weight requirement. The weight requirement for an empty reservoir may be close to the weight of the reservoir without any fluid therein such that, e.g., the system can confirm that the new reservoir is empty. The weight requirement for a new full reservoir may be close to the weight of the reservoir when it is full of fluid such that, e.g., the system can confirm that the new reservoir contains a full, or complete, amount of fluid. After the reservoir has been calibrated (e.g., weighted) and determined ready for use, the calibration notification may be dismissed.

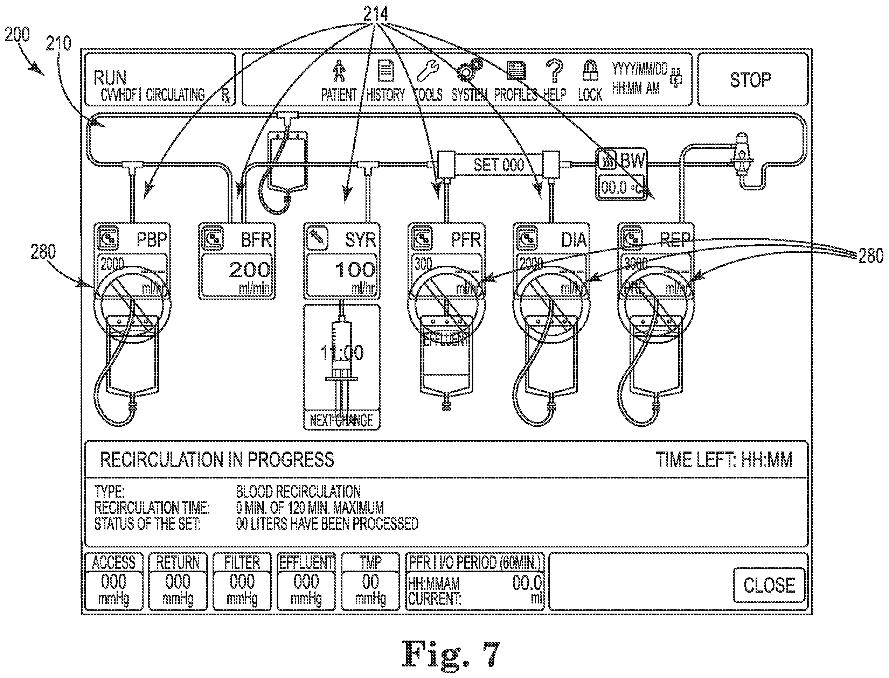

In the case where a user, or operator, is recirculating the fluid in an extracorporeal circuit temporarily in the absence of a patient, a stop notification 280, e.g., a circle with a slash through it, greying out, darkening, color changing, or dulling the fluid area, and/or any other visual indication to indicate a pause or stoppage , may be displayed proximate a fluid area 214 (e.g., over one or both of the pump element and the reservoir element) as shown in FIG. 7. The stop notifications 280 may indicate to a user which pumps are stopped during recirculation. In other words, when a pump for a fluid is paused, a stop notification 280 may be displayed proximate a fluid area corresponding to the fluid. Additionally, after the pumps resume operation (e.g., after the recirculation as completed), the stop notifications 280 may be dismissed from the fluid areas 214.

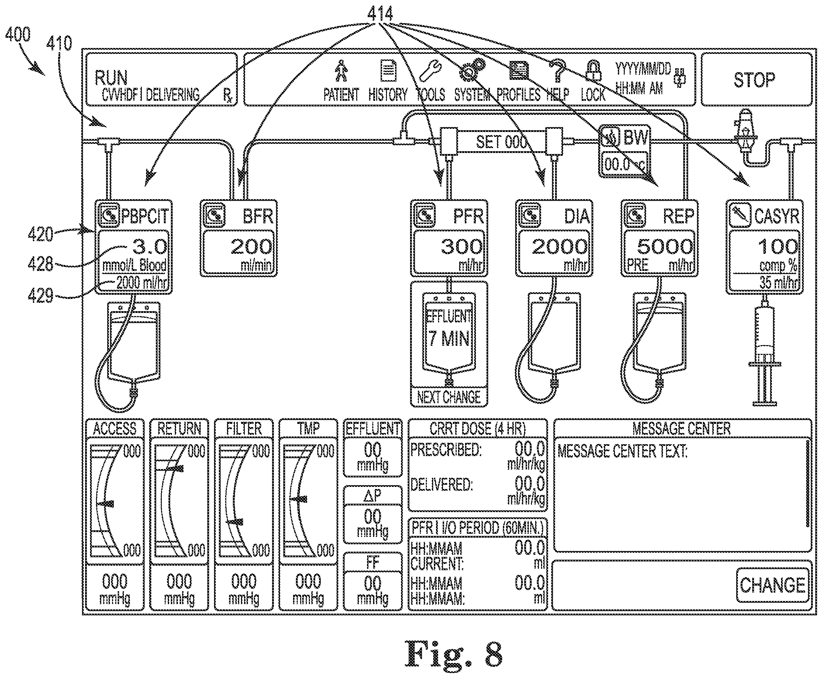

Another exemplary graphical user interface 400 including a fluid region 410 is depicted in FIG. 8. As shown, the fluid region 410 includes a plurality of fluid areas 414 similar to the fluid areas 214 described herein with reference to FIGS. 2-4 but in a different treatment configuration or arrangement.

A pump element 420 may further include a dose concentration 428 and a flow rate 429 as shown in FIG. 8. In the case of a citrate solution provided on a pre-blood pump scale, the citrate dose in millimoles per liter of blood (mmol/L Blood) may be of primary concern to a user, and thus, may be displayed as the dose concentration 428 while the flow rate may be displayed as the flow rate 429.

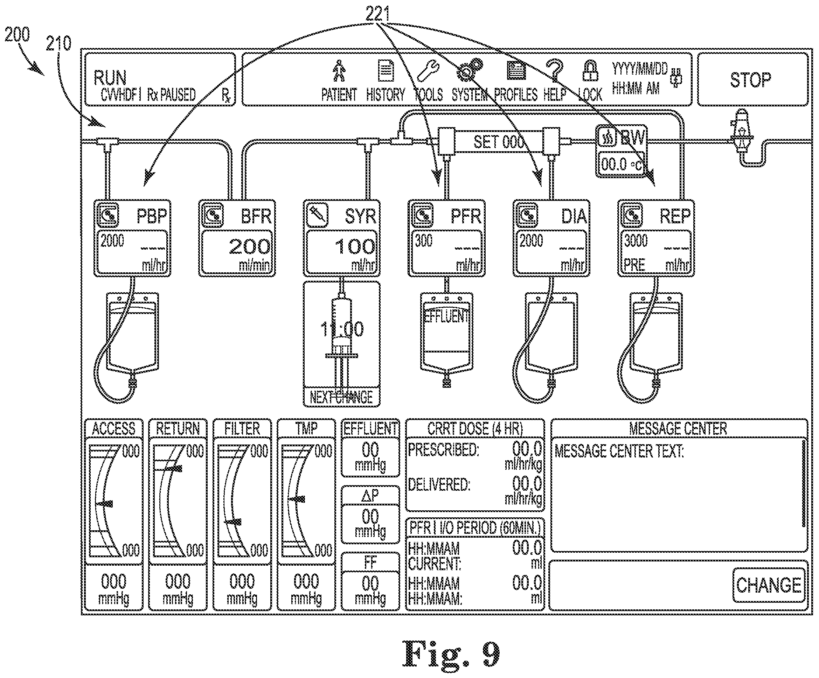

Blood treatments may be paused for various reasons. During a paused blood treatment, the exemplary graphical user interface 200 may be displayed as shown in FIG. 9. When a treatment is paused, one or more pumps may be paused, or turned off, and thus, the flow rate of the one or more paused pumps may be zero. As such, the pump elements of the fluid areas 221 corresponding to paused pumps may be indicated as being paused. For example, as shown in FIG. 9, if a pump's flow rate is zero (during a paused treatment), the pump element of fluid areas 221 may be displayed as translucent and the flow rates may be depicted as zero as shown by dashes. Additionally, the old flow rates may be depicted in the upper left corners of the pump elements of the fluid areas 221 to, e.g., indicate to a user what the last flow rates were before the pause in treatment. Of course, any indications may be used.

## Claims

1. An extracorporeal blood treatment system comprising:
a display apparatus (22) comprising a graphical user interface (200; 300; 400), wherein the graphical user interface (200; 300; 400) is configured to depict a fluid region;
pumps (120; 220; 320; 420) configured to move blood and a treatment solution during extracorporeal blood treatment; and
a computing apparatus (12) operatively coupled to the display apparatus (22), **characterized in that** the apparatus further comprises:
reservoir scales (130), wherein each reservoir scale of the reservoir scales is configured to weigh a reservoir (132; 230; 330) operably attached to the reservoir scale;
**and in that** the computing apparatus (12) is configured to:
display on the graphical user interface (200; 300; 400) a fluid region comprising a plurality of fluid areas, wherein each fluid area of the plurality of fluid areas (214; 314; 414) corresponds to a fluid, and is representative of a pump of the pumps configured to pump the fluid represented by the fluid area and one reservoir scale of the reservoir scales configured to weigh the fluid, wherein each fluid area of the fluid areas (214; 314; 414) comprises a pump element and a reservoir element, wherein the pump element defines a flow rate of the fluid and the reservoir element comprises a fluid level representative of an amount of the fluid within a reservoir storing the fluid, wherein the reservoir element comprises a graphical depiction of a fluid stored in the reservoir, and wherein the fluid level representative of the amount of fluid within the reservoir is defined by the level of fluid graphically depicted in the reservoir element;
receive a weight signal from each reservoir scale (130), with the weight signal from each reservoir scale (130) being indicative of the weight of a reservoir (132; 230; 330) attached to the reservoir scale (130);
make a determination that a reservoir (132; 230; 330; 430) attached to a reservoir scale (130) from which the weight signal has been received has passed a selected weight limit at least partially based on the weight signal received from the reservoir scale (130); and
display a next reservoir change notification (240; 350) proximate a fluid area of the one or more fluid areas (214; 314; 414) configured to indicate that the fluid within a reservoir is the next fluid that requires a reservoir change, wherein the next reservoir change notification (240; 350) comprises a time period representing an amount of time left before the reservoir change is required.

2. The system of claim 1, wherein the plurality of fluid areas corresponds to one or more of pre blood pump, effluent, citrate, blood flow rate, patient fluid removal, dialysate, replacement fluid, anticoagulation, patient plasma loss, and calcium.

3. The system of any one of claims 1 and 2, wherein the reservoir element comprises a graphical depiction of a fluid stored in a fluid bag, and wherein the fluid level representative of the amount of fluid within the reservoir is defined by the level of fluid graphically depicted in the fluid bag.

4. The system of any one of the preceding claims, wherein the computing apparatus (12) is further configured to execute:
displaying on the graphical user interface (200; 300; 400) a change region;
allowing a user to select the change region to initiate one of a flow rate change and a bag change; and
displaying a time period representing an amount of time left before the reservoir change is required proximate a plurality of fluid areas of the one or more fluid areas.

5. The system of any one of the preceding claims, wherein each fluid area of the one or more fluid areas (214; 314; 414) comprises a fluid connection extending between the pump element and the reservoir element.

6. The system of any one of the preceding claims , wherein the computing apparatus (12) is further configured to execute:
displaying a stop notification proximate a fluid area of the one or more fluid areas (214; 314; 414) when a pump for the fluid is paused; and
dismissing the stop notification if the pump resumes operation.

7. The system of any one of the preceding claims , wherein the computing apparatus (12) is further configured to execute:
displaying a changing reservoir notification proximate a fluid area of the one or more fluid areas (214; 314; 414) when a reservoir for the fluid is being changed; and
dismissing the changing reservoir notification if the reservoir is removed.

8. The system of any one of the preceding claims, wherein the computing apparatus (12) is further configured to execute:
displaying a calibration notification proximate a fluid area of the one or more fluid areas (214; 314; 414) when a reservoir for the fluid is being calibrated by the extracorporeal blood treatment system; and
dismissing the calibration notification if the reservoir is finished being calibrated and ready for use.

9. The system of claim 1, wherein the extracorporeal blood treatment system further comprises
one or more reservoir status lights, wherein each reservoir status light of the one or more reservoir status lights is associated with one reservoir scale of the one or more of reservoir scales, and wherein the one or more reservoir status lights comprises a first reservoir status light associated with a first reservoir scale of the one or more reservoir scales and a second reservoir status light associated with a second reservoir scale of the one or more reservoir scales, and further wherein the first reservoir status light emits light from a location that is closer to the first reservoir scale than the second reservoir scale.

## Patentansprüche

1. Extrakorporales Blutbehandlungssystem umfassend:
eine Anzeigeeinrichtung (22) umfassend eine graphische Benutzerschnittstelle (200; 300; 400), wobei die graphische Benutzerschnittstelle (200; 300; 400) konfiguriert ist zum Darstellen einer Fluidregion;
Pumpen (120; 220; 320; 420), die konfiguriert sind zum Bewegen des Blutes und einer Behandlungslösung während der extrakorporalen Blutbehandlung; und
eine Recheneinrichtung (12), die mit der Anzeigeeinrichtung (22) operativ verbunden ist,
**dadurch gekennzeichnet, dass** die Einrichtung weiter umfasst:
Behälterwaagen (130), wobei jede Behälterwaage der Behälterwaagen konfiguriert ist zum Wiegen eines an der Behälterwaage operativ befestigten Behälters (132; 230; 330) ;
und dadurch, dass die Recheneinrichtung (12) konfiguriert ist zum:
Anzeigen auf der graphischen Benutzerschnittstelle (200; 300; 400) einer Fluidregion umfassend eine Vielzahl von Fluidbereichen, wobei jeder Fluidbereich der Vielzahl von Fluidbereichen (214; 314; 414) einem Fluid entspricht und eine Pumpe der Pumpen, die zum Pumpen des durch den Fluidbereich dargestellten Fluids konfiguriert sind, und eine Behälterwaage der zum Wiegen des Fluids konfigurierten Behälterwaagen darstellt, wobei jeder Fluidbereich der Fluidbereiche (214; 314; 414) ein Pumpelement und ein Behälterelement umfasst, wobei das Pumpelement eine Durchflussmenge des Fluids definiert und das Behälterelement einen Fluidstand umfasst, der eine Menge des Fluids in einem das Fluid enthaltenden Behälter darstellt, wobei das Behälterelement eine graphische Darstellung eines im Behälter enthaltenen Fluids umfasst, und wobei der Fluidstand, der die Fluidmenge im Behälter darstellt, durch den im Behälterelement graphisch dargestellten Fluidstand definiert ist;
Empfangen eines Gewichtssignal von jeder Behälterwaage (130), wobei das Gewichtssignal von jeder Behälterwaage (130) das Gewicht eines an der Behälterwaage (130) befestigten Behälters (132; 230; 330) angibt;
Feststellen, dass ein Behälter (132; 230; 330; 430), der an einer Behälterwaage (130), von der das Gewichtssignal empfangen wurde, befestigt ist, eine ausgewählte Gewichtsgrenze überschritten hat, mindestens teilweise basierend auf dem von der Behälterwaage (130) empfangenen Signal; und
Anzeigen einer Folgebehälteränderungsmeldung (240; 350) in der Nähe von einem Fluidbereich der einen oder mehreren Fluidbereiche (214; 314; 414), die konfiguriert ist zum Angeben, dass das Fluid in einem Behälter das folgende Fluid ist, das eine Behälteränderung braucht, wobei die Folgebehälteränderungsmeldung (240; 350) eine Zeitspanne umfasst, die eine restliche Zeitmenge darstellt, bevor die Behälteränderung erforderlich wird.

2. System nach Anspruch 1, wobei die Vielzahl von Fluidbereichen einem oder mehreren von Vorblutpumpe, Abfluss, Citrat, Blutdurchflussmenge, Fluidentfernung vom Patienten, Dialysat, Substitutionsfluid, Antikoagulation, Plasmaverlust des Patienten und Calcium entspricht.

3. System nach einem der Ansprüche 1 und 2, wobei das Behälterelement eine graphische Darstellung eines in einem Fluidbeutel enthaltenen Fluids umfasst, und wobei der die Fluidmenge im Behälter darstellende Fluidstand durch den im Fluidbeutel graphisch dargestellten Fluidstand definiert ist.

4. System nach einem der vorherigen Ansprüche, wobei die Recheneinrichtung (12) weiter konfiguriert ist zum:
Anzeigen auf der graphischen Benutzerschnittstelle (200; 300; 400) einer Änderungsregion;
Erlauben, einem Benutzer, die Änderungsregion auszuwählen, um entweder eine Durchflussmengenänderung oder eine Beuteländerung zu starten; und
Anzeigen einer Zeitspanne, die eine restliche Zeitmenge darstellt, bevor die Behälteränderung erforderlich wird, in der Nähe einer Vielzahl von Fluidbereichen der einen oder mehreren Fluidebereiche.

5. System nach einem der vorherigen Ansprüche, wobei jeder Fluidbereich (214; 314; 414) eine Fluidverbindung umfasst, die sich zwischen dem Pumpelement und dem Behälterelement erstreckt.

6. System nach einem der vorherigen Ansprüche, wobei die Recheneinrichtung (12) konfiguriert ist zum:
Anzeigen einer Stoppmeldung in der Nähe von einem Fluidbereich der einen oder mehreren Fluidbereiche (214; 314; 414), wenn eine Fluidpumpe gestoppt ist; und
Verwerfen der Stoppmeldung, wenn die Pumpe den Betrieb wieder aufnimmt.

7. System nach einem der vorherigen Ansprüche, wobei die Recheneinrichtung (12) konfiguriert ist zum:
Anzeigen einer Änderungsbehältermeldung in der Nähe von einem Fluidbereich der einen oder mehreren Fluidbereiche (214; 314; 414), wenn ein Fluidbehälter geändert wird; und
Rücksetzen der Änderungsbehältermeldung, wenn der Behälter entfernt wird.

8. System nach einem der vorherigen Ansprüche, wobei die Recheneinrichtung (12) konfiguriert ist zum:
Anzeigen einer Kalibrierungsmeldung in der Nähe von einem Fluidbereich der einen oder mehreren Fluidbereiche (214; 314; 414), wenn ein Fluidbehälter vom extrakorporalen Blutbehandlungssystem kalibriert wird; und
Verwerfen der Kalibrierungsmeldung, wenn die Kalibrierung zu Ende ist und der Behälter gebrauchsfertig ist.

9. System nach Anspruch 1, wobei das extrakorporale Blutbehandlungssystem weiter umfasst
eine oder mehrere Behälterstatuslampen, wobei jede Behälterstatuslampe der einen oder mehreren Behälterstatuslampen einer Behälterwaage der einen oder mehreren Behälterwaagen zugeordnet ist, und wobei die eine oder mehrere Behälterstatuslampen eine erste Behälterstatuslampe, die einer ersten Behälterwaage der einen oder mehreren Behälterwaagen zugeordnet ist, und eine zweite Behälterstatuslampe, die einer zweiten Behälterwaage der einen oder mehreren Behälterwaagen zugeordnet ist, umfassen, und weiter wobei die erste Behälterstatuslampe Licht von einer Stelle, die der ersten Behälterwaage näher ist als der zweiten Behälterwaage, emittiert.

## Revendications

1. Système de traitement extracorporel du sang comprenant :
un appareil de visualisation (22) comprenant une interface utilisateur graphique (200; 300; 400), où l'interface utilisateur graphique (200; 300; 400) est configurée pour illustrer une région de fluide;
des pompes (120; 220; 320; 420) configurées pour déplacer le sang et une solution de traitement pendant le traitement extracorporel du sang ; et
un appareil de calcul (12) couplé de façon opérationnelle à l'appareil de visualisation (22),
**caractérisé en ce que** l'appareil comprend en outre :
des balances pour réservoirs (130), où chaque balance pour réservoirs est configurée pour peser un réservoir (132; 230; 330) attaché de façon opérationnelle à la balance pour réservoirs;
et **en ce que** l'appareil de calcul (12) est configuré pour :
visualiser sur l'interface utilisateur graphique (200; 300; 400) une région de fluide comprenant une pluralité de zones de fluide, où chaque zone de fluide de la pluralité de zones de fluide (214; 314; 414) correspond à un fluide et est représentative d'une pompe des pompes configurées pour pomper le fluide représenté par la zone de fluide et une balance pour réservoirs des balances pour réservoirs configurées pour peser le fluide, où chaque zone de fluide des zones de fluide (214; 314; 414) comprend un élément de pompe et un élément de réservoir, où l'élément de pompe définit un débit du fluide et l'élément de réservoir comprend un niveau de fluide représentatif d'une quantité du fluide dans un réservoir contenant le fluide, où l'élément de réservoir comprend une illustration graphique d'un fluide contenu dans le réservoir, et où le niveau de fluide représentatif de la quantité de fluide dans le réservoir est défini par le niveau de fluide illustré graphiquement dans l'élément de réservoir ;
recevoir un signal de poids de chaque balance pour réservoirs (130), le signal de poids de chaque balance pour réservoirs (130) étant indicatif du poids d'un réservoir (132; 230; 330) attaché à la balance pour réservoirs (130);
établir qu'un réservoir (132; 230; 330; 430) attaché à une balance pour réservoirs (130), de laquelle le signal de poids a été reçu, a dépassé une limite de poids sélectionnée basée au moins partiellement sur le signal de poids reçu de la balance pour réservoirs (130) ; et
visualiser une notification de changement de réservoir successif (240; 350) proche d'une zone de fluide de l'une ou plusieurs zones de fluide (214; 314; 414), configurée pour indiquer que le fluide dans un réservoir est le fluide successif nécessitant un changement de réservoir, où la notification de changement de réservoir successif (240; 350) comprend une période de temps représentant une quantité de temps résiduelle avant qu'il est nécessaire de changer le réservoir.

2. Système selon la revendication 1, où la pluralité de zones de fluide correspond à une ou plusieurs pompes pré-sang, effluent, citrate, débit du sang, élimination de fluide du patient, dialysat, fluide de substitution, anticoagulation, perte de plasma du patient et calcium.

3. Système selon l'une quelconque des revendications 1 et 2, où l'élément de réservoir comprend une illustration graphique d'un fluide contenu dans une poche de fluide, et où le niveau de fluide représentatif de la quantité de fluide dans le réservoir est défini par le niveau de fluide illustré graphiquement dans la poche de fluide.

4. Système selon l'une quelconque des revendications précédentes, où l'appareil de calcul (12) est configuré en outre pour :
visualiser sur l'interface utilisateur graphique (200; 300; 400) une région de changement ;
permettre à un utilisateur de sélectionner la région de changement pour faire commencer l'un entre un changement de débit et un changement de poche ; et
visualiser une période de temps représentant une quantité de temps résiduel avant qu'il est nécessaire de changer le réservoir proche d'une pluralité de zones de fluide de l'une ou plusieurs zones de fluide.

5. Système selon l'une quelconque des revendications précédentes, où chaque zone de fluide de l'une ou plusieurs zones de fluide (214; 314; 414) comprend une liaison de fluide s'étendant entre l'élément de pompe et l'élément de réservoir.

6. Système selon l'une quelconque des revendications précédentes, où l'appareil de calcul (12) est configuré en outre pour :
visualiser une notification d'arrêt proche d'une zone de fluide de l'une ou plusieurs zones de fluide (214; 314; 414) lorsque une pompe pour le fluide est en pause ; et
annuler la notification d'arrêt si la pompe reprend son fonctionnement.

7. Système selon l'une quelconque des revendications précédentes, où l'appareil de calcul (12) est configuré en outre pour :
visualiser une notification de changement de réservoir proche d'une zone de fluide de l'une ou plusieurs zones de fluide (214; 314; 414) lorsque un réservoir pour le fluide est changé ; et
annuler la notification de changement de réservoir si le réservoir est retiré.

8. Système selon l'une quelconque des revendications précédentes, où l'appareil de calcul (12) est configuré en outre pour :
visualiser une notification de calibration proche d'une zone de fluide de l'une ou plusieurs zones de fluide (214; 314; 414) lorsque un réservoir pour le fluide est calibré par le système de traitement extracorporel du sang ; et
annuler la notification de calibration si la calibration du réservoir est terminé et qu'il est prêt à l'emploi.

9. Système selon al revendication 1, où le système de traitement extracorporel du sang comprend en outre
un ou plusieurs voyants d'état des réservoirs, où chaque voyant d'état des réservoirs de l'un ou plusieurs voyants d'état des réservoirs est associé à une balance pour réservoirs de l'une ou plusieurs balances pour réservoirs, et où l'un ou plusieurs voyants d'état des réservoirs comprennent un premier voyant d'état des réservoirs associé à une première balance pour réservoirs de l'une ou plusieurs balances pour réservoirs et un deuxième voyant d'état des réservoirs est associé à une deuxième balance pour réservoirs de l'une ou plusieurs balances pour réservoirs, et en outre où le premier voyant d'état des réservoirs émet de la lumière d'une position plus proche de la première balance pour réservoirs que de la deuxième balance pour réservoirs.
